# EUROPEAN PATENT APPLICATION

(11) **EP 2 444 549 A1**
(43) Date of publication of application: **25.04.2012**
(21) Application number: 10188174.6
(22) Date of filing: 20.10.2010
(51) Int. Cl.: D06M 16/00, D06M 17/04, A61K 38/46, A61L 15/18, A62D 5/00, B32B 27/12, D06N 7/00, D06M 10/02, D06M 15/564, D06M 23/04

(54) **Textile materials with bioactive protection**

(71) Applicant: Stazione Sperimentale per la Seta, 20133 Milano (IT); MASCIONI S.p.A., I-20123 Milano (IT); Centro Tessile Cotoniero e Abbigliamento S.p.A., 21052 Busto Arsizio (VA) (IT); Cittadini S.p.A., 25050 Paderno Franciacorta, Brescia (IT)
(72) Inventor: Isella, Francesca, I-20045, Besana Brianza Monza E Brianza (IT); Rosace, Giuseppe, I-24044, Dalmine Bergamo (IT); Giovannoni, Gianluigi, I-21030, Orino, VARESE (IT); Donelli, Ilaria, I-20099, Sesto San Giovanni Milano (IT); Alberti Fusi, Gabriella, I-22100, Como (IT); Cittadini, Cesare, I-25050, Paderno Franciacorta Brescia (IT); Freddi, Giuliano, I-20133, Milano (IT)
(74) Representative: Long, Giorgio

(57) **Abstract**

The present invention relates to textiles endowed with bioactive protection against air or water borne toxic chemicals such as pesticides, nerve agents, phenols, polycyclic aromatic hydrocarbons, etc. More specifically, the object of the invention is a textile material carrying immobilized biocatalysts able to actively degrade toxic chemicals adsorbed onto it. The textile material of the invention can be used to manufacture bioactive interlining element of protective garments or it may, but not exclusively, be used was the bioactive layer of filter masks, gloves and other protective devices. In particular, the present invention relates to a bioactive textile material comprising (i) a textile support; (ii) a bioactive layer either on one or both aides of the said textile support, the bioactive layer comprising a nanostructured xerogel film which comprises an immobilized bloactive enzyme.

## Description

### Filed of the invention

The present invention relates to textiles endowed with bioactive protection against air or water borne toxic chemicals such as pesticides, nerve agents, phenols, polycyclic aromatic hydrocarbons, etc. More specifically, the object of the invention is a textile material carrying immobilized biocatalysts able to material carrying immobilized biocatalysts able to actively degrade toxic chemicals adsorbed onto it. The textile material, of the invention can be used to manufacture bioactive interlining element of protective garments or it may, but not exclusively, be used as the bioactive layer of filter masks, gloves and other protective devices.

### Background art

Occasional or persistent exposure to variable levels of toxic agents in accidentally/voluntarily contaminated environments or in working places may be very harmful for workers and professionals, as well as for common people who may accidentally incur dangerous situations during their every-day life. Nowadays, the development of effective means to protect people from the risks of exposure to toxic agents has become a key issue. Clothing has acted as a second skin for human beings since pre-historic times, having fulfilled primary protective and insulating functions towards the outside environment, before becoming also a mean of cultural identity and communication. For these reasons, clothing still represents the most effective way to realize protection concepts.

Barrier protection is the state-of-the-art technology for protective clothing. Layers of active charcoal integrated into textiles result in high, non specific absorbing capacity towards a wide range of harmful chemicals. These textiles typically provide passive protection, just preventing the contact of chemicals with skin and mucosa. However, the barrier technology is frustrated by some drawbacks, such as bulkiness and heaviness of textiles, lack of comfort for the wearer during practical use, saturation effect (adsorption is an equilibrium process whose rate and extent depend on various parameters, among others the morphology of the adsorbent and the concentration of the adsorbate), problems of disposal of contaminated textiles (the toxic agent is present in concentrated form in its still active state).

The concept of active protection against toxic agents, associated with that of self-decontamination, has recently been introduced by some authors who integrated biological catalysts (enzymes) into textiles via a range of enzyme immobilization and stabilization technologies (US 7,270,373 B2; US 7,343,169 B1; US 2009/0325261 A1). Enzymes able to catalyze the hydrolysis of organophosphate compounds and other toxic agents, i.e. organophosphorous hydrolase (OPH), organophosphorous acid anhydrolase (OPAA), and other hydrolases, have been investigated for their immobilization onto textile surfaces in order to reader them bioactive and effective for decontamination. Challenges in developing effective protection/decontamination systems are the preservation of a high catalytic activity of the immobilized biocatalysts under storage and operative conditions and the accessibility of the embedded enzyme to the toxic agents. Stability and accessibility issues have been faced by adopting various integrated approaches, such as the multilayer deposition of polyelectrolytes by the Layer-by-Layer (LbL) technology and the non covalent immobilization of the enzyme on the surface of polymerized vesicles (liposomes) or silica particles.

Bioactive protective textiles developed until now provide a range of advantages against passive barrier protection systems because enzymes specifically and effectively attack and degrade harmful compounds, thus avoiding the accumulation of toxic agents onto the textile material itself and minimizing the concerns about disposal of depleted protective goods. However, these systems cannot be effective over an extended period or time (including both storage and use times) because enzymes inevitably undergo a gradual loss of activity during storage, use and maintenance of the textile onto which they are immobilized. The rate of activity decay can be different depending on the kind of enzyme, on the immobilization technology, on the storage and use conditions, on the exposition to environmental stresses, etc. Moreover, maintenance operations, such as domestic or industrial washing cycles, are likely to affect not only the stability of the catalyst itself but also the durability of the polymeric immobilization system.

Based on the aforementioned advantages any drawbacks of existing protective textiles, there is an urgent need for developping novel systems able to combine the specificity and effectiveness of the bioactive protection concept, with a higher stability and durability of the protective textiles under storage, use and maintenance conditions, as well as with the innovative multi-use concept which is first introduced with the present invention as a tool to fulfil medium-to-long term application requirements.

### Summary of the invention

The present invention describes a process designed to integrate enzymes onto textile materials in order to endow the said textile material with a bioactive protection against chemical agents toxic for human beings and the environment such as pesticides, nerve agents, phenols, polycyclic aromatic hydrocarbons, etc. Enzyme classes of interest for the invention comprise, but are not limited to, hydrolases and oxidoreductases, which have demonstrated the ability to degrade a wide range of toxic agents. Enzymes can be used individually or in combination of two, three or more of them to ensure multifunctional performance.

In case of depletion of bioactivity as a consequence of use and maintenance operations or natural decay, the bioactive protective function can be refreshed/reloaded during washing of protective textiles in professional laundries, thus applying the concept of rechargeability.

The invention is directed preferably, but not exclusively, to textile materials, in form of woven, knitted, or nonwoven fabrics. These can be made of natural or manmade fibres (artificial, synthetic) or their blends. The bioactive textile material resulting from the process of the invention will be subsequently incorporated into protective clothing systems or devices like masks, filters, etc, as an independent interlining element that can be detached and treated separately for maintenance and rechargeability purposes.

The bioactive textile material of the invention, according to an embodiment, has a layered structure comprising the textile support, a microporous polymeric layer and a nanostructured bioactive film with the enzyme immobilized into it.

In an embodiment, the textile support is activated at the surface level by means of chemical or physical systems, preferably but not exclusively by vacuum plasma treatment, in order to enhance adhesion of chemicals.

The surface of the textile support is coated with a microporous polymeric foam in order to enhance the specific surface area, to maximize the load of enzyme per unit area and to achieve the optimum level of air permeability. Finally, the bioactive layer is applied as nanosol loaded with the enzyme, which is transformed into a xerogel film upon thermal curing.

Further characteristics and the advantages of this invention will be better understood from the following detailed description of some embodiments thereof, which are provided by way of non-limiting examples.

### Brief description of the drawings

Figure 1: Peptidase activity assay. The intensity of absorbance at 405 nm is plotted as a function of reaction time; after 1 min the reaction attains a plateau.

Figure 2 :Organophosphorous activity assay. Emission Spectra of Coumaphos in blank (curve a, OPH = 0 µg/ml) and test (curve b, OPH 33 µg/ml; curve c, OPH = 66 µg/ml) samples. Fluorescence curves recorded after 25 min incubation. Excitation was set at 254 nm.

Figure 3: Activity of peptidase immobilized onto a PA fabric as afunction of curing température and time

Figure 4: Activity of peptidase immobilized onto a PA fabric as a function of the storage time.

Figure 5: Activity of peptidase immobilized onto a PA fabric as a function of the number of washing cycles.

Figure 6: Activity of peptidase immobilized onto a PA fabric before and after reloading A: starting sample; B: sample A after 16 months storage under ambient conditions; C: sample A after 5 washings; B1: sample B after bioactivity recharging; C1: sample C after bioactivity recharging.

Figure 7: Morphological properties of foam coated fabrics. A optical microscopy image of foam coated Vilene fabric; B: scanning electron microscopy image of the surface of a foam coated CO/PA fabric; C: crosssection image of the foam coating of sample B; D: close up of image C with pore size measures of image C with pore size measures.

Figure 8: Assay of OPH activity after immobilization onto a foam coated textile support (Vilene fabric). Emission spectra of 0,04 mM Coumaphos before (curve a)and after contact with the bioactivated fabric (curves b-d, recorded at 15 min 20 min and 25 min after incubation, respectively). Excitation was set at 254 nm.

Figure 9: Intensity of the fluorescence emission at 464 nm of 0,04 mM Coumaphos solutions exposed to different bioactivated fabrics for different times 15,20, and 25 min). The fluorescence intensity of the degradation products formed by OPH catalyzed hydrolysis of Coumaphos increased with the time of incubation.

### Detailed description of the invention

The invention relates to bioactive textile materials comprising
(i) a textile support, preferably in form of fabric, optionally pre-treated with. O₂ plasma;
(ii) a bioactive layer consisting of to nanostructured xerogel film comprising an immobilized bioactive enzyme.

According to an embodiment, the invention relates, to bioactive textile materials comprising (i) a textile support, preferably in form of fabric, optionally pre-treated with O₂ plasma; (ii) a microporous polymeric foam coating; (iii) a bioactive layer consisting of a nanostructured xerogel film comprising an immobilised bioactive enzyme,

The invention further relates to a method for preparing the said bioactive textile materials.

In an embodiment, the textile support is preliminarily activated by oxygen plasma treatment.

In an embodiment, the microporous polymeric foam coating is apt to modulate air permeability and to provide a large surface area for the adhesion of the bioactive layer.

In an embodiment, the nanostructured xerogel film is formed via sol-gel process,

In an embodiment, the steps leading to bioactivation of textile materials are as follows:
- Selection of the textile support and of the enzyme or enzyme mix according to end-use requirements,
- Optionally, plasma activation of the textile support on one or both surfaces,
- Foam coating of one or both surfaces of the optionally plasma activated textile support,
- Preparation of the nanosol containing the enzyme (s) ,
- Application of the bio-loaded nanosol by impregnation and formation of a lyogel onto the of the foam-coated textile support,
- Transformation of the lyogel into a nanostructured xerogel film with the enzyme immobilized into it by thermal curing
- Optionally, detection of enzyme(s) activity.

The textile support can be a woven, knitted or nonwoven fabric made of natural (cotton, wool, silk, etc.) mineral (glass ceramics, etc.) or manmade fibres. (polyester, polyamide, polyacrylonitrile, polypropylene, aramids, etc.) or their blends.

Composite fabric comprising yarn reinforcements made of metal yarn, glass, carbon fibres or organic polymer materials such a aramids are also included.

Other composite Materials, polymer membranes and nanostructured matrices comprising nanofibres produced via the electrospinning technology are suitable supports for the process of the invention. The selection of the appropriate textile or textile composite support is driven by the targeted end-use application. Here we refer to current knowledge of the structure/function/performance relationship in the field of technical textiles, which is rapidly evolving under the pressure of market demand for functional, high-tech, engineered protective textile materials.

Enzymes of interest for the invention are biocatalysts able to interact with target molocules of potentially toxic agent and to degrade them into non-toxic by-products. Commercially available enzymes, endowed with high stability towards chemical and physical stresses, preferably but not exclusively belonging to the hydrolases and oxidoreductases classes, are suitable for the purpose of the invention. Examples of preferred enzymes include organophosphorous hydrolase (EC 3.1.8.1), organophosphorous acid anhydrolase (EC 3.1.8.2), laccase (EC 1.10,3.2), peroxidase (EC 1.11.1.7). These enzymes are capable of interacting with and degrading various toxic molecules, including nerve agents, pesticides, phenolic contaminants, etc,

Other suitable enzymes can be hydrolases acting on peptide bonds (peptidases; EC 3.4) and on ester bonds (EC3 3.1) which are active against biological polymers constituting living organisms like viruses and bacteria. All these enzymes can be used alone or in binary, ternary, etc. combination to widen the bioactivity range. Commercial enzyme products are supplied in form of granules, powder or aqueous solution. Before use, the biocatalyst activity is tested by means of specific tasting methods provided by the supplier or available from the scientific and technical literature. This allows determining the activity per unit weight or volume necessary to define the enzyme concentration in the application medium.

The plasma treatment is a well known technology able to generate a wide range of active chemical species onto the surface of polymeric, textile materials. These active chemical species, mostly radicals, can be exploited to enhance adhesion and binding strength of chemicals and polymers applied onto textiles by exhaust impregnation or coating technologies as surface finishing agents. Vacuum plasma treatment under reduced atmosphere, of oxygen gas has been used here to activate the textile support but also atmospheric plasma treatments in the presence of air or argon or other inert gases can be used for the sake of the process of the invention.

Coating textile materials with polymers is a widely applied technology in the field of technical textiles. The coating process consists in the application of a viscous liquid, usually an aqueous solution of a thermoplastic polymer, onto the surface of a textile material, Followed by drying or curing processes. After evaporation of the solvent, a solid, film is formed whose morphological chemical, physical and mechanical properties, as well as performance and durability are influenced by the kind of polymer and the coating technology used. Polyvinyl chloride (PVC) , polyvinylidene. chloride (PVDC), polytetrafluoroethylene (PTFE), natural rubber, styrene-butadiene rubber (SBR), silicon rubber, polyurethane and other polymers are mostly used for coating technical textiles. Selection depends on required end-use performance in terms of gas permeability transparency, abrasion., weathering, flame resistance, water and oil repellence, chemical and thermal stability, tensile strength, resilience, easy of processing, etc.

Polyurethanes (PUs) are the preferred coating polymers for the invention. PUs are made by reaction of a diisocyanate with a diol. The versatile chemistry of PUs allows a wide range of coating options to be realized. PUs for textile application are commercially available preparations supplied in form of stable prepolymers able to react at elevated temperatures in the presence of a catalyst. Coating techniques depend on whether the textile material has to be coated on one or both sides. Impregnation techniques based on an impregnating trough followed by a passage through a couple of squeezing rollers are adequate for two sides coating. If coating has to be made only on one side or two different coatings have to be made on each side, other technique must be used such as lick roll coating, knife coating, gravure coating, etc.

PUs for foam coating of textile substrates have to meet a series of key application requirements in terms of adhesion onto natural and synthetic fibres, stability to light, weathering and aging, flexibility, porosity, compatibility with other organic and inorganic components of coating recipes, etc.

In a preferred embodiment of this invention the selected PU is an aqueous dispersion of polyester/polyether polyurethane with aliphatic polyol and polyisocyanate moieties. The anionic character is attributed to PU preferably by the presence or free carboxylic groups. Preferred physical-chemical properties are: pH 7-10, specific gravity 1.0-1.2 g/cm², solid content 40-60%. Compatibility with acrylate based thickeners, finishing auxiliaries (pigment pastes, flame retardants, antimicrobial, softeners, etc.), and low sensitivity to salts are other preferred characteristics.

The PU dispersion must be foamed in rotor-type mixers at 200-600 g/L, preferably 300-500 g/L, after mixing with acrylate-based thickener in an amount to attain a viscosity preferably of 10-20 dPas as measured by a rotational viscometer.

To enhance the stability of the final coating a cross-linking agent, for example an alkyl-modified melamine resin cross-linkable at about 140-160°C, can be added to the PU formulation in an amount of 0,1-5% of the polymer weight.

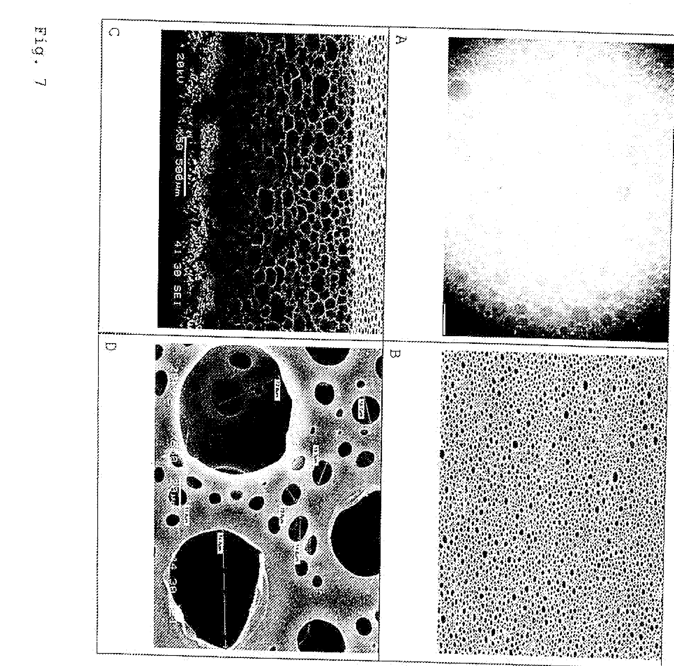

An even microporosity is the most important parameter for the performance of the PU foam coating of the present embodiment. A microporous foam is defined as the one having a high density microcellular structure with large size pores preferably falling in the range 50-150 µm, medium size pores preferably in the range 10-50 µm, and small size pores preferably in the range 1-10 µm. In this kind of microcellular structure the large and medium size pores are interconnected by the small size pores formed onto the surface of the polymeric walls which limit the pore volume (see: Figure 7). This cellular structure is obtained by adding appropriate surfactants, preferably is obtained by adding appropriate surfactants, preferably ammonium/alkyl amine stearate surfactants, added in an amount corresponding to 1-15% of the PU weight, preferably 5-10%, and by adjusting the pH at 9-10 with ammonia.

Application of formulated PU foam is normally carried out by knife-over-roller coater or cylinder or rotary screen. The thickness of the coating is in the 0,1-1 mm range, preferably 0,3-0,7 mm. Drying is done at 80-90°C, calendaring is optional, and curing is done at 140-160°C for 1-5 min. The final coating must ensure high elastic recovery and good mechanical properties. Typical elongation at break values must fall in the 500-800% range.

A xerogel according to the invention is a colloidal solid obtained by drying a gel at temperatures generally comprised between 80°C and 140°C. The xerogel has a high porosity (up to 50% v/v of voids) and a very high surface (150-900 m²/g), with pores of 1-10 nm size.

The xerogel according to the invention can be produced by the so called sol-gel process.
The sol-gel process is a well known technique used for the deposition of thin ceramic films onto the surface of polymers, including textile materials. Silica or other inorganic nanosols can be produced by either acid- or alkali-catalysed hydrolysis of the corresponding silicon or metal alkoxides in water or any organic solvent miscible with water. Under suitable conditions nanosols undergo polymerization through condensation thus forming three-dimensional nanostructured aggregates. The sol-gel matrix can be chemically modified by co-hydrolysis and co-condensation with, for example, alkoxysilanes (with an organic residue R = alkyl) to produce organically modified silica. This approach allows matrix variation and a number of sol-gel systems for textile application has already been made commercially available.

Sol-gel materials having high extent of hydrophilicity coupled to a high level of compatibility towards entrapped biological molecules, such as those produced with biocompatible silane precursors and by aqueous processing methods that involve removal of alcohol by-products by evaporation before the addition of biomolecules, are preferred for the sake of the invention.

The sol-gel nanosystems can effectively incorporate biological compounds. The reasons of the interest for this kind of bio-composites are as follows:
(i) they offer a unique approach, to exploit biological structures for industrial uses;
(ii) the preparation can be accomplished under mild conditions which allow the use of thermally labile substances;
(iii) inorganic materials as host offer unique mechanical, thermal and chemical stability for preserving biological molecules;
(iv) the porosity of these nanoparticulate matters and the density and degree of immobilization of biological molecules can be finely tuned for the intended application. Silica-derived ceramic matrices possess the additional advantage of being toxicologically and biologically inert thus ensuring the viability of encapsulated bioactive substances.

Large biomolecules like enzymes are non diffusible when incorporated into the sol-gel nanosystem. The immobilization of enzymes into these nanostructured materials takes big advantages from the high surface area of the nanomaterial, thus leading to improved enzyme loading and increased stability. Nanostructured materials offer many advantages compared to traditional immobilization techniques because the shape and the size of the substrate can be controlled at the nanometre scale and the range of available approaches to permanently bind and to stabilize an enzyme is very wide.

According to the invention, a nanosol precursor, such as the ones describes above, is added with an amount of the bioactive enzyme, then this composition is used to of the bioactive enzyme, then this composition is used to coat the textile material. After drying, the coated textile material is cured at temperatures between 80°C and 140ºC 30 sec-20 min to form a xerogel loaded with the bioactive enzyme.

The nanosol precursor may be a commercial product which is usually employed in the textile industry.

In an embodiment, the volume ratio of bioactive enzyme in the nanosol precursor will be between 1:1 and 1:10, preferably between 1:3 and 1:7, more preferably between 1:3 and 1:5.

The choice of curing temperature in the formation of the xerogel is critical. Higher curing temperatures improve the mechanical characteristics of the xerogel coating and the preservation of the enzymatic activity upon time and washing cycles. On the other hand, several bioactive enzymes are temperature sensitive, so that a too high temperature may cause an initial decay of the enzymatic activity. An optical balance between these requirements must be reached and setting of the curing temperature will depend on the enzyme that is used and on the application to which the textile material is devoted.

According to an embodiment, the curing temperature for obtaining the xetogel is between 90ºC and 110°C,preferably between 95ºC and 105ºC.

The xerogel coating can be applied directly to the textile surface or to the microporous polymeric foam coating as described above.

An important advantage of the method of the invention is that the whole process leading to the preparation of bioactive: textile materials can be executed by using currently available textile technologies which bring fluids in close contact with the fibres forming the textile material thus favouring adsorption, adhesion and physical or chemical anchoring of the active ingredients contained in the fluid onto the fibre surface. These technologies include but are not limited to batch processes, where both the fluid and the textile material move with respect one to the other or only one of them (the fluid or the textile material) moves and the other is stationary, as well as continuous processes generally consisting in the impregnation of the textile material with a high concentrated solution of the processing aids made by padding, coating or spraying, followed by fixation and drying steps.

Another advantage is that in case of depletion of bioactivity as a consequence of natural decay or use and maintenance operations, the bioactive protective function or the textile material can be refreshed during a normal washing cycle. The bioactive textile material of the invention, is intended to be used to manufacture bioactive interlining element as part of protective garments or, but not exclusively, it becomes the bioactive layer of filter masks, gloves and other protective devices. In the case of protective garments, the lining elements is separated and subjected to a maintenance cycle with the aim to wash out by-products accumulated during previous uses and restore the bioactivity lost as a consequence of use and maintenance stresses. Afterwards, the lining elements are assembled again to the garment.

Only for illustrative purposes, examples of preparation and testing textile materials with bioactive protection are given here.

### EXAMPLE 1

### Assay of the enzymatic activity immobilized onto textile supports.

### Peptidase Assay

Samples of fabrics with immobilized peptidase activity were tested for their ability to hydrolyze N-Succinyl-Ala-Ala-Pro-Phe p-Nitroanilide according to a modified published procedure (Sigma Enzymatic Assay). The reaction follows the scheme:
N-Succinyl-Ala-Ala-Pro-Phe p-Nitroanilide + H₂O → N-Succinyl-Ala-Ala-Pro-Phe + p-nitroanilide

The formation of p-nitroanilide was detected spectrophotometricaly at 405 nm.

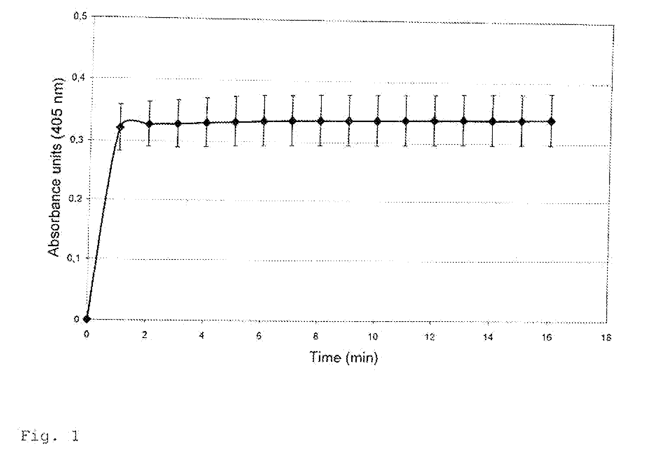

A 20 mM stock solution of the N-Sccnyl-Ala-Ala-Pro-Phe p-Nitroanilide peptide was prepared in DMSO. Then, the test solution was prepared by adding 10 µl of the stock solution to 990 µl of 0.1 M potassium phosphate buffer at pH 8 containing 10 mM CaCl₂ and 0.005% Tween 80. Two strips of 1.5 x 8 cm were cut from a sample of fabric with immobilized peptidase and laid onto the surface of a testplate of a Varioskan Flash Multimode Reader (Thermo Scientific), Four drops, 13 µl each of the test solution were pipetted onto the surface of one strip at regular distances. Four drops of water were pipetted onto the surface of the other strip (blank). The testplate was immediately inserted into the Varioskan reading unit and absorbance values at 40:5 nm were recorded for each drop of blank and test samples every 20 sec, over a total reading time of 5 min. It was observed that the absorbance values increase steadily until 1 min after deposition of the drop and then attain a plateau value remaining constant up to 5. min (Figure 1). Thus the absorbance values recorded at 405 nm after 1 min incubation were used to express the activity of the immobilized peptidase.

### Organophosphorous hydrolase Assay

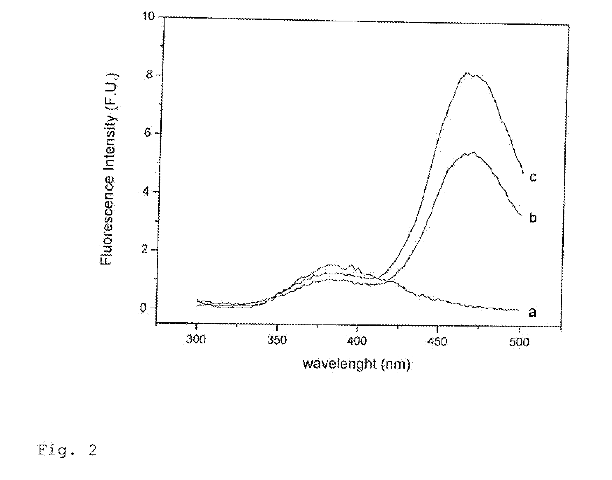

Samples of fabrics with immobilized organophosphorpous hydrolase activity were tested for their ability to hydrolyze 3-chloro-7-diethoxyphosphinothioyloxy-4-methyl-2-chromenone (Coumaphos, Sigma), a non-volatile, fat-soluble organophosphate able to kill insects and mites. The 1 mM stock solution of Coumaphos was prepared in acetonitrile. Then, the test solution was prepared by adding 40 µl of the Coumaphos stock solution to 960 µl of 50 mM Tris-HCl buffer at pH 8 containing 0.1 M CoCl₂. Two pieces of 1 x 1 cm dimension were cut from a sample of fabric with immobilised organophosphorous hydrolase. One piece was immersed in 1 ml of the test solution another piece was immersed if 1 ml of butter (blank). The two samples were incubated at 45ºC for 15 min. Afterwards, 250 µl of solution were taken from the reaction vial every 5 min and pipetted into a well of a Varioskan testplate for fluorescence measurement. Excitation was set at 254 nm. The emission spectrum was recorded in the range 300-500 nm (Figure 2). The emission peak at 385 nm corresponds to the Coumaphos substrate, while that at 464 nm to the emission. of the degradation product formed by hydrolysis with organophosphorous hydrolase. After about 25-30 min from the beginning of incubation the reaction attained a plateau as indicated by the constancy of the fluorescence emission value. The value of fluorescence emission at 464 nm was used to express the activity of immobilized organophosphorous hydrolase

### EXAMPLE 2

*Deposition stabilization of the nanostructured bioactive film on a textile support*

Two plain woven fabrics, one made of 100% cotton (CO) and the other made of 100% polyamide (PA) were used as textile support for the deposition of the nanostructured film containing the immobilised enzyme Samples of the size of a A4 sheet were cut from the fabrics and used for the tests. The enzyme was a peptidase (subtilising; 3.4.21.62) supplied by Novozymes under the commercial name Alcalase 2.5L. The nanosol precursor was a commercial organic-inorganic colloidal suspension used for the treatment of textile surfaces via the sol-gel process (iSys MTX, Italian). To a 10-20 g/L, preferably 15 g/L of iSys: MTX at pH 5-6 a fixed amount of peptidase was added so that the peptidase:nanosol volume ratio was in the range 1:3-1:5. After mixing, the solution was poured into the trough of a laboratory scale paddrying machine. Samples of CO and PA fabric were impregnated with a 2-roll podder Mathis Type HVF until a wet pick up of about 50 w% and then cured with a steamer unit Mathis Type DH. Curing temperatures and times were varied in the 80- 140°C and 1-10 min range, respect Afterwards, the enzynatic of the immobilized peptidase was assayed as described in Example 1.

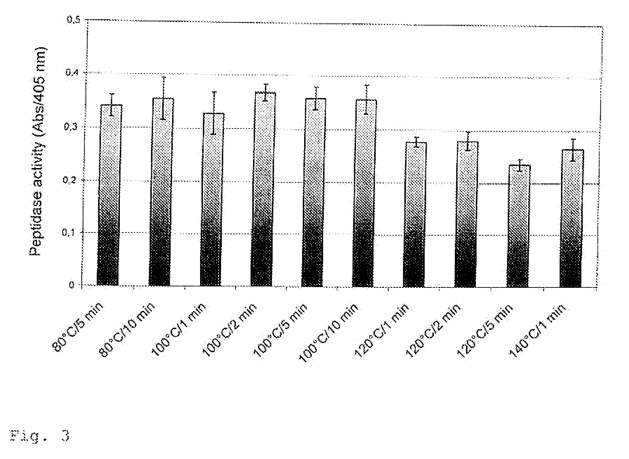

Figure 3 shows the activity values of peptidase immobilised onto PA fabric as a function of curing temperature and time. No significant changes were detected among samples for a curing temperature ≤100°C increasing the curing temperature up to 140°C the activity decreased but still reached about 75% of the level of activity of the best performing samples. The post-coating thermal treatment is necessary to obtain a xerogel film with good mechanical properties, stability and durability With increasing temperature and time of the annealing step these properties improved. However, care must be taken to preserve the integrity of thermally labile biological molecules like enzymes. The results illustrated in Figure. 3 show that the best compromise between costing stability and enzyme activity can be found over a wide range of temperatures and times.

### EXAMPLE 3

Shelf life stability and durability of bioactive textiles

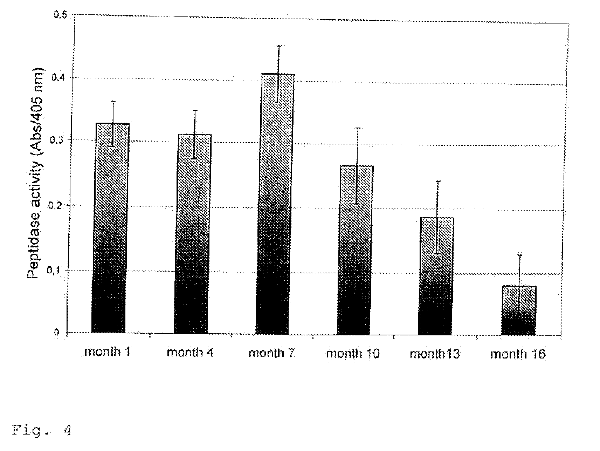

Samples of PA fabrics were treated as described in EXAMPLE 2. Curing temperature and time were 100°C and 10 min, respectively. The bioactive textile material 15 with immobilized peptidase were tested for peptidase activity soon after preparation according to the assay method of EXAMPLE 1 and then were stored under normal ambient conditions. The peptidase assay was repeated regularly over a period of 16 months. The results are shown in Figure 4. The peptidase activity remained at highly level 1 until month 10, afterwords it started decreasing gradually until month 16. The significantly higher value recorded at month 7 can be attributed to a higher ambient temperature (summer season) which enhanced the enzyme activity. The long shelf life stability of the bioactive textile materials of the invention fits the time scale from processing of the textile material itself, to manufacture of the protective garment or device, until its use in the field.

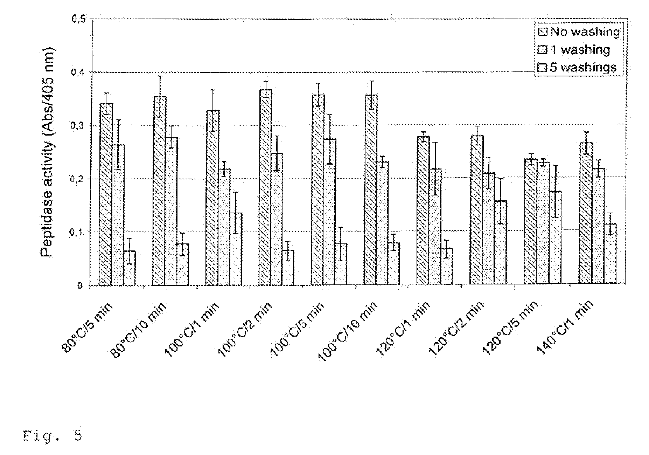

To investigate the durability of the nanostructured bioactive film under maintenance conditions, the PA fabric samples with immobilized peptidase activity prepared as described in EXAMPLE 2 were washed according to ISO 6330:2000 standard testing method. The peptidase activity was tested according to the assay method of EXAMPLE 1 after sample preparation and then after 1 and 5 washings. The results are shown in figure 5. Washing resulted ion a decrease of the peptidase activity, which was more evident after 5 washing cycles. This effect can be attributed either to teaching of enzyme: from the nanostructured film or to worsening of enzyme activity due to the adverse effect of washing chemicals and conditions or to a concomitant effect of both factors. Interestingly, the samples cured at 120° for 2 and 5 min displayed a proportionally lower decrease of peptidase activity, taking the activity of the respective non washed samples as a reference. After 5 washings a these samples retained a peptidase activity higher than that of the other samples cured at lower temperature,

Thus, it; can generally be inferred that the higher the curing temperature, the higher the stability of the nanostructured film the more durable the enzyme activity under maintenance conditions.

### EXAMPLE 4

*Reloading of decayed bioactivity*

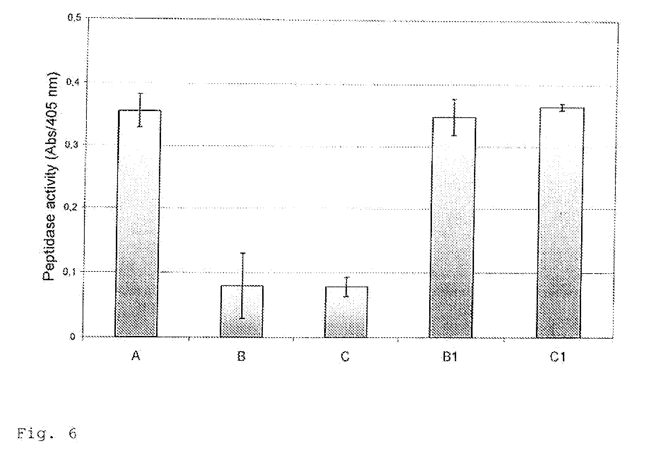

As experienced from the samples of EXAMPLE 3, the bioactivity may undergo more or less extensive decay as a consequence of storage or use and maintenance operations, samples of PA fabrics with immobilized peptidase activity (figure 6, Sample A) were prepared as described in example 3 and stored for 16 months under ambient conditions (Figure 6, Sample B) or washed 5 times (Figure 6.. Sample C) Peptidase activity was assayed according to the method described, in Example 1. Then, fabric samples A and B were subjected to enzyme reloading: in an after-washing cycle carried out in a beaker dyeing machine Labomat Mathis under the following conditions: temperature 45ºC, time 15 min,agitation 30 rpm, material-to-liquor ratio: 1:50, peptidase:nanosol volume ratio 1: 4 (Example 2) rinsing with running water and oven drying at 100^{º}C for 2 min. Afterwards, samples were test for peptidase activity. The results shown in Figure 6 for samples B1 and C1 show that the initial biöcatalytic activity was fully, recovered by means of the reloading procedure performed.

### EXAMPLE 5

*Deposition of microporous polymeric foam coating onto a textile support pre -treated with O₂ plasma*

Two kinds of fabrics were used for the tests:
(i) a plain weave 50% cotton/PES fabric (CO/PA; 166 g/m²);
(ii) a Vilene PFT 101 nonwoven fabric (Freudenberg, Vliesstoffe KG -: Interlining Division, Weinheim, Germany), of 6.6 g/m² weight and 0.7mm thickness, made of aramidic fibres. Before foam coating, both fabrics were treated with O₂ plasma under reduced vacuum, (50-100 Pa) with a Vacuum Plasma machine model KPR-180, equipped with RF generator-operating at 10 KHz, current 200 A fabric speed 10-15 m/min.

In an embodiment of this invention CO/PA and vilene fabrics were coated on one side with 150-250 g/L of Tubicoat Mp (CHT Italia S.r.l.), an anionic aqueous PU dispersion able to form a microporous foam layer onto textile substrates. Coating thickness was 0.3-0,7 mm, preferably 0,5 min. The PU layer was dried at 110ºC and pressed between, rubber cylinders at 4 bar. The other side of the fabrics was coated in three variants: (i) with 200 400 g/L, preferably 300 g/L polymer preparation Tubicoat WLI (CHT Italia S.r.l.) applied as unstable foam; (ii) with Tubicoat. WLI (CHT Italia S.r.l.) diluted with 50% water, thickened and applied by means of a air squeegee; (iii) with 300-500 g/L polymer preparation Tubiscreen EX-TS (CHT Italia S.r.l) applied as unstable foam. After coating, all samples were cured at. 150°C for 3 min

In another embodiment of this invention plasma treated CO/PA and Vilene fabrics were coated on one side with 200-400g/L, preferably 250-300g/L of Dicrylan PGS (Huntsman), an anionic aliphatic polyester/ether PU dispersion in water, as stable foam coating with thickness of 0,5-1 mm Drying was performed at 100°C followed by calendaring at 80ºC, 2 atm. An unstable foam coating was applied onto the other side of the fabrics comprising 50-200 g/L, preferably 80-120 g/L Dicrylan PSF (Huntsman), an anionic aqueous preparation of modified polyether urethane, or 100-20:0 g/L, preferably 150 g/L Invasan RCD (Huntsman), a cross-linkable polymer preparation containing reactive cyclodextrins. Drying was done at 90°c and then fabrics were subjected to polymerization: at 150-160º)C

Foam coated CO/PA and Vilene fabrics were characterized for weight/unit area and air permeability. The weight increased from 166 9/m² to 280-330 g/m² for the CO/FA Fabric, and from 66 g/m² to 140-170 g/m² for the Vilene fabric. Air permeability (10 mm) decreased from 14 mm/sec to 8-10 mm/sec for the CO/PA fabric, and from 1500 mm/sec to 100-200 mm/sec for the Vilene TNT fabric.

Coated fabrics were also characterised by optic and scanning electron microscopy to provide evidence of the shape and size of the interconnected network of micropores formed at the fabric surface by foam coating with the different PU preparations (Figure 7).

### EXAMPLE 6

*Activation of the foam coated textile with nanostructured bioactive film*

Samples of foam coated CO/PA and ViLene fabrics prepared and characterised as described in Example 5 were treated with a bioactive nanosol containing organophosphorous hydrolase as described in EXAMPLE 2. Curing temperature and time were 100ºC and 5 min, respectively. The bioactive textile materials with immobilized peptidase were then tested for hydrolytic activity against Coumaphos according to the assay method of EXAMPLE 1

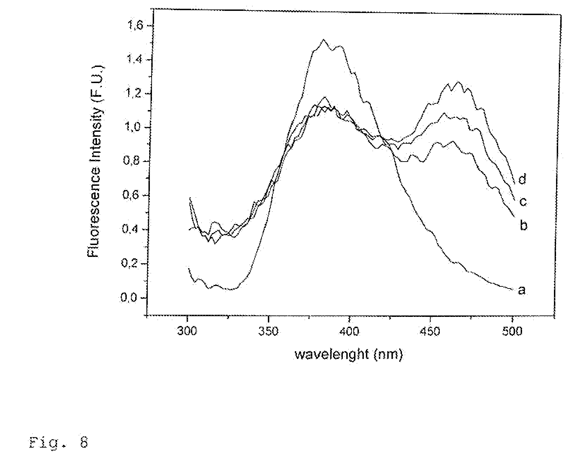

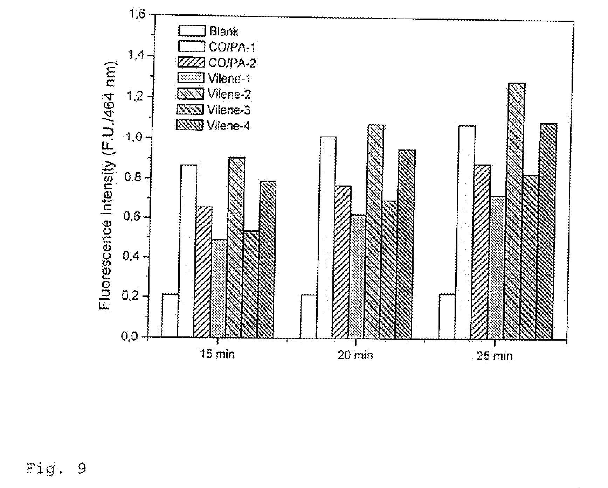

The results reported in Figure 8 show typical emission fluorescence spectra of Coumaphos before (curve a, emission peak at 385 nm) and After contact with the bioactivated fabric (curves b-d, emission peaks at 385. nm and 464 nm for the residual non hydrolysed Coumaphos and hydrolysis products, respectively). Figure.9 shows that all the fabric samples with immobilised organophosphorous hydrolase were bioactive against the Coumaphos substrate. The rate and extent of degradation was mostly related to the thickness of the foam coating i.e. to the extent of loading of the bioactivated nanosol preparation. In this respect the influence of the texture of the fabric support was irrelevant.

## Claims

1. A bioactive textile material comprising (i) a textile support; (ii) a bioactive layer either on one or both sides of the said textile support, the bioactive layer comprising a nanostructured xerogel film which comprises an immobilized bioactive enzyme.

2. The bioactive textile material of claim 1,comprising (i) a textile support; (ii) a microporous polymeric foam coating either on one or both sides of said textile support; (iii) a bioactive layer on said microporous polymeric foam coating, the said bioactive layer consisting of a nanostructured xerogel film comprising an immobilised bioactive enzyme,

3. The bioactive textile material of claim 1 or claim 2, wherein the textile support is preliminarily activated by plasma treatment.

4. The bioactive textile material of any claim 1 to 3, wherein the said textile support is a woven, knitted or nonwoven fabric made of natural (cotton, wool, silk, etc.), mineral (glass, ceramics etc.) or manmade fibres. (polyester, polyamide, polyacrylonitrile, polypropylene, aramids, etc.) or their blends, or a composite fabric comprising: yarn reinforcements made of metal yarn, glass, carbon fibres or organic polymer materials such as aramids, or nanofibres produced via the electrospinning technology.

5. The bioactive textile material of any claim 1 to 4, wherein the bioactive enzyme is able to interact with target molecules of potentially toxic agent and to degrade them into non-toxic by-products.

6. The bioactive textile material of claim 5, wherein the bioactive enzyme is selected among hydrolases and oxidoreductases.

7. The bioactive textile material of claim 6, wherein the bioactive enzyme is selected in the group consisting of organophosphorous hydrolase (EC 3.1. 8.1), organophosphorous acid anhydrolase (EC 3.1,8.2), laccase (EC 1.10.3.2), peroxidase (EC 1.11,1.7), hydrolases acting on peptide bonds (peptidases;EC 3.4) and on ester bonds (E'C 3.1) and mixtures thereof. ,

8. The bioactive textile material of any claim 2 7, wherein the microporous polymer foam is selected among polyvinyl chloride (PVC), polyvinylidene chloride (PVDC), polytetrafluoroethylene (PTFE), natural rubber, styrene-butadiene rubber (SBR), silicon rubber, polyurethane (PU) microporous foams.

9. The bioactive textile material of claim 8, wherein the said: polyurethane is an aqueous dispersion of polyester/polyether polyurethane with aliphatic polyol and polyisocyanate moieties having free carboxylic groups.

10. The bioactive textile material of claim 9, wherein the polyurethane is cross-linked with an aikyl-modified melamine resin in an amount of 0,1-5% of the polymer weight.

11. The bioactive textile material of clam 9 or claim 10, which has a high density microcellular structure with large size pores falling in the range 50-150 µm, medium
size pores in the range 10-50 µm, and small size pores in the range 1-10 µm .

12. The bioactive textile material of claim 11, comprising ammonium/alkyl amine stearate Surfactants in an amount corresponding to 1-15% of the polyurethane weight, or in an amount corresponding to 5-10% of the polyurethane weight.

13. The bioactive textile: material according to any claim 2 to 12, wherein the microporous polymer foam coating has a thickness in the 0,1-1 mm range, or in the 0,3-0,7 mm range.

14. The bioactive textile material of any claim 1 to 13, wherein the nanostructured xerogel has a porosity or up to 50% v/v of voids and a surface of 150-900 m²/g, with pores of 1-10 nm size.

15. The bioactive textile material of any claim 1 to 14, wherein the said xerogel is obtainable by the hydrolysis of a silicon or metal alkoxide nanosol precursor, optionally with alkoxysilanes,in the presence of said bioactive enzyme, and by post-drying curing the said nanosol at a temperature selected in the range of 80-140ºc.

16. The bioactive textile material according to claim 15, wherein the volume ratio of bioactive enzyme in the nanosol precursor is between 1:1 and 1:10, or between 1:3 and 1:7 or between 1:3 and 1:5.

17. process for manufacturing a bioactive textile material according to any claim 1 to 16, comprising the following steps:
a) Selection of the textile support and of-the enzyme or enzyme mix according to end-use requirements,
b) Optionally, plasma activation of the textile support on one or both sides,
c) Microporous polymer foam coating of one or both slides of the optionally plasma activated textile support,
d) Preparation of a nanosol precursor containing the enzyme or a mixture of enzymes to obtain an enzyme-loaded nanosol precursor,
e) Application of the enzyme -loaded nanosol precursor by impregnation and formation of a lyogel onto the surface of the foam-coated textile support,
f) Transformation of the lyogel into a nanostructured Xerogel film with the enzyme immobilized into it by thermal curing.

18. The process of claim 17, wherein the said step b) of plasma activation is performed by vacuum plasma treatment of the textile support under reduced atmosphere of oxygen gas.

19. The process of claim 17 or claim 18, wherein the step c) of microporous polymer loam coating is performed by impregnation followed by a passage through squeezing rollers for two sides coating, or lick roll coating, knife coaling or gravure coating for coating on one side or different coatings for the two sides, or by knife-over-roller coater or cylinder or rotary screen.

20. The process according to any claim 17 to 19, wherein the step c) of microporous polymer foam coating comprises foaming a polyurethane aqueous dispersion in a rotor-type mixer at 200-600 g/L, preferably 300-500 g/L, after mixing with acrylate-based thickener in an amount to attain a viscosity preferably of 10-20 dPas as measured by a rotational viscometer.

21. The process of claim 20, wherein the said-polyurethane aqueous dispersion is cross -Linked with an alkyl-modified melamine resin, in an amount of 0,1-5% of the polymer weight, at a temperature selected in the range of 140-160°C.

22. The process of claim 20 or claim 21, wherein the said polyurethane aqueous dispersion is added with ammonium/alkyl amine stearate surfactants in an amount corresponding to 1-15% of the ployurethane weighty, preferably 5-10% of the ployurethane weight, at a pH of 9-10.

23. The process or any claim 17 to 22, wherein the said microporous polymer foam coating is dried at 80-90ºC, optionally calendared and finally cured at 140-160ºC for 1-5 min.

24. The process of any claim 17 to 23, wherein the said steps d) to f) are performed according to the sol-gel process.

25. The process of any claim 17 to 24, wherein step f) is performed at temperatures between 80ºC and 140ºC for 30 sec-20 min to form a xerogel loaded with the bioactive enzyme.

26. The process of claim 25, wherein the step f) is performed, at temperatures between 90ºC and 110°C_{,} or between 95ºC and 105ºC_{.}
